# EUROPEAN PATENT SPECIFICATION

(11) **EP 0 522 567 B1**
(45) Date of publication and mention of the grant of the patent: **02.10.1996**
(21) Application number: 92111730.5
(22) Date of filing: 10.07.1992
(51) Int. Cl.: G01L 9/00, A61B 5/0215

(54) **Pressure converter**
Druckwandler
Convertisseur de pression

(30) Priority: 12.07.1991 JP 172201/91; 16.07.1991 JP 175170/91; 09.06.1992 JP 149313/92
(43) Date of publication of application: 13.01.1993
(73) Proprietor: TERUMO KABUSHIKI KAISHA, Tokyo (JP)
(72) Inventor: Kudo, Takeshi, Ashigarakami-gun, Kanagawa-ken (JP); Komatsu, Kiyoshi, Ashigarakami-gun, Kanagawa-ken (JP); Kitamura, Takashi, Ashigarakami-gun, Kanagawa-ken (JP)
(74) Representative: Casalonga, Axel

(56) References cited:
- EP-A- 0 180 662
- EP-A- 0 360 286
- GB-A- 2 207 804
- US-A- 4 815 472
- US-E- 33 518

## Description

This invention relates to a pressure converter to be used in the medical field. More particularly, it relates to a disposable pressure converter to be used for the determination of vital blood pressure, intrauterine pressure, intravesical pressure, intrasophageal pressure, and other specific pressures.

In recent years, the outbreak of a problem of infection with viruses responsible for hepatitis and aids has been urging the development of a disposable pressure converter in the medical field. Among the pressure converters of this kind known to the art is counted that which is disclosed in EP-A-0360286 (1990).

In this pressure converter, a semiconductor sensor chip is provided with a diaphragm for exposure to pressure. This semiconductor sensor chip is fastened with adhesive agent to the surface of an insulating substrate, which has a thick-film resistor formed by the printing treatment. The semiconductor sensor chip and the thick-film resistor are electrically interconnected with a lead wire. The insulating substrate is provided with an electrical feed through terminal capable of establishing electrical continuity between the obverse and reverse sides of the substrate. A retainer plate is provided with a connecting terminal adapted for contact with the electrical feed through terminal. This connecting terminal is electrically connected to an electric cable by being soldered thereto. To the insulating substrate, a cylindrical coupling lid is attached with adhesive agent in such a manner as to encircle the semiconductor sensor chip. This coupling lid is filled in the interior thereof with a pressure transmitting medium (such as, for example, silicone resin). This pressure transmitting medium serves the purpose of electrically insulating the semiconductor sensor chip from the fluid subjected to determination of pressure and of preventing the alkali metals (such as Na⁺ and K⁺) and alkaline earth metals (such as Ca²⁺) possibly contained in the fluid under test from soiling or adhering to the semiconductor sensor chip. A sensor assembly comprising the component parts mentioned above is set in place in a through hole intercommunicating between a sensor accommodating chamber and a fluid chamber formed in a housing.

In this pressure converter, the pressure of the fluid for test held inside the fluid chamber is transmitted to the diaphragm part of the semiconductor sensor chip through the medium of the pressure transmitting medium. The diaphragm part of the semiconductor sensor chip bends in under the pressure and varies the resistance of a pressure sensitive gauge formed by the treatment of thermal diffusion on the surface of the diaphragm part. The electrical signal which is generated in response to this variation of the pressure is transmitted to an external display or recording device through an electric cable laid via the lead wire, thick-film resistor, electrical feed through terminal, and connecting terminal.

In the conventional pressure converter constructed as described above, however, the pressure transmitting medium of the constitution of gel filling the interior of the coupling lid has caused various problems. Since the pressure transmitting medium is interposed between the fluid under test and the semiconductor sensor chip, for example, it gives rise to the phenomenon of damping during the process of pressure transmission and renders accurate detection of the pressure waveform difficult. Since the semiconductor sensor chip and the coupling lid are fastened to the insulating substrate with adhesive agent, the possibility exists that the adhesive agent will be unevenly applied and the consequent inferior adhesion will cause the pressure transmitting medium in the form of gel to leak through the joints of poor adhesion and obstruct accurate determination of pressure. The possibility also arises that the interior of the- coupling lid will be filled uniformly with the pressure transmitting medium only with difficulty and the unevenly filled pressure transmitting medium will suffer occurrence therein of bubbles and consequently obstruct accurate determination of pressure. And the packing of the pressure transmitting medium cannot be adapted in the manufacturing process of the semiconductor sensor by a batch process utilizing a semiconductor integrated circuit manufacturing technology, so there is a problem,that the conventional pressure converter is low in productibility. Further, there are cases when the pressure transmitting medium will burst into the fluid chamber while it is undergoing the treatment of sterilization (autoclaving).

The conventional pressure converter uses an expensive insulating substrate such as, for example, an alumina substrate or a ceramic substrate which has a thick-film resistor printed thereon as a seat for the semiconductor sensor chip but also comprises numerous component parts such as the connecting terminal, coupling lid, and pressure transmitting medium. Moreover, for the purpose of adjusting the offset output signal, each of the pressure converters is required to have the thick-film resistor thereof subjected to laser trimming. Thus, the cost of material and the cost of production are both so high as to render supply of inexpensive pressure converters difficult.

Further, in the conventional pressure converter, since the semiconductor pressure sensor and the coupling lid are fastened with adhesive agent to the insulating substrate, the possible inferior adhesion due to uneven application of the adhesive agent entails leakage of the pressure transmitting medium through the points of poor adhesion and renders accurate determination of pressure unattainable.

EP-A-180 662 discloses a measuring transducer comprising a pressure sensor chip provided with a deformable silicon diaphragm, and integrated strain sensitive resistors.

US -A-4 815 472 discloses a multipoint pressure-sensing catheter system comprising a silicon transducer chip having a thin centrally located diaphragm.

GB-A-2 207 804 discloses a semiconductor diaphragm pressure sensor comprising a substrate and piezo-resistors isolated from the diagphram by an insulating layer, in order to overcome the problem of electrical leakage and to allow the sensor to function effectively at temperatures well in the excess of 150°C. The formation of said insulating layer consists in facing in close contact two bodies and causing then growth of the oxide layer.

An object of this invention is to provide an inexpensive pressure converter which obviates the necessity for interposing a pressure transmitting medium of the form of gel between the semiconductor pressure sensor chip and the pressure. fluid under test, avoids various problems such as damping encountered by the conventional pressure converter, allows a generous decrease in the number of component parts, enjoys simplicity of construction, decreases the cost of material and the cost of production, and obtains stable outputs.

Another object of this invention is to provide an inexpensive pressure converter of high quality which absorbs the ununiformity of pressure retaining force possibly suffered to occur when a pressure retaining lid is fitted in a pressure retaining lid fitting part, avoids exerting undue strain or stress on the sensor, and allows a decrease in the offset voltage.

A further object of this invention is to provide a pressure converter which allows the formation of a protective film intended for preventing the staining due to contact of the pressure fluid under test with a semiconductor pressure sensor chip to be incorporated in the batch process utilizing the technique for the production of a semiconductor integrated circuit and warrants inexpensive and volume production and a method for the production of the pressure converter.

As claimed, the invention proposes a pressure converter comprising a semiconductor pressure sensor including a sensor substrates, a diaphragm part formed on said sensor substrate and adapted to deform proportionately to the pressure emanating from a pressure medium under test, a gauge resistor adapted to vary the magnitude of resistance thereof proportionately to the deformation of said diaphragm part, wherein at least the pressure-receiving surface of said diaphragm part is coated with a silicon oxynitride protective film.

According to an embodiment of the invention, the converter further comprises a fluid pipe adapted to allow flow of the pressure fluid therein and is provided in the lateral wall thereof with a pressure detecting hole, the pressure-receiving surface of the diaphragm part being opposed to the the pressure detecting hole.

The opposite pressure-receiving surfaces of said diaphragm part can be coated with a protective film.

In the pressure converter of this invention, since the pressure-receiving surface of the semiconductor pressure sensor chip is coated with the dielectric protective film, the semiconductor pressure sensor chip does not directly contact the pressure fluid under test and, as a result, the sensor chip is prevented from being stained as with an alkali metal, is enabled to enjoy an improved electrical insulating property and possesses ideal temperature characteristics. Since this pressure converter does not require, as does the conventional countertype, the pressure transmitting medium of the form of gel to be interposed between the sensor chip and the pressure fluid under test, the problems of pressure loss due to the formation of bubbles in the pressure transmitting medium and the occurrence of damping during the process of pressure transmission are completely eliminated. Thus, the pressure converter brings about an effect of enabling accurate detection of pressure waveform free from distortion strain and loss of sharpness. Of course, the possibility of the pressure transmitting medium bursting while the pressure converter is undergoing the process of sterilization is also nil.

The pressure converter according to an embodiment of this invention is further provided with pressing means for pressing the periphery of the pressure-receiving surface of the semiconductor pressure sensor chip against the outer wall surface of the fluid pipe and sealing means for sealing watertightly the gap between the outer wall surface of the. fluid pipe and the semiconductor pressure sensor chip. Specifically, the pressing means is composed of a pressing lid fitting part formed as projected along the periphery of the pressure detecting hole in the fluid pipe and a pressing lid fitted into the pressing lid fitting part, provided with a protuberant part, and adapted to press the semiconductor pressure sensor chip against the outer wall surface of the fluid pipe by virtue of the protuberant part. The sealing means can be composed of a groove part formed in the outer wall surface of the fluid pipe in such a manner as to encircle the pressure detecting hole and an annular elastic member inserted in the groove part and pressed by the protuberant part of the pressing lid through the medium of the semiconductor pressure sensor chip.

In the essential components of the conventional pressure converter, i.e. the semiconductor pressure sensor chip, the seat of insulating plate, and the coupling lid, the pressure converter of this invention finds use only for the semiconductor pressure sensor chip as an indispensable component and obviates the necessity for the other two components. The sealing method using the annular elastic member (0-ring) of highly reliable performance is adopted for sealing the gap between the sensor chip and the outer wall of the fluid pipe, i.e. the sole site in need of fast union. This construction completely obviates the necessity for joining parts with adhesive agent, decreases one of the causes for imperfect sealing, warrants highly reliable sealing infallibly with the O-ring, further realizes a decrease in the number of component parts, and permits a generous decrease in the cost of material and the cost of assembly.

Further, according to another embodiment of this invention the pressure converter permits a generous decrease in the number of component parts, simplifies the construction of itself, allows an ample reduction in the cost of material and the cost of assembly, enjoys overall economization with the preclusion of a work for laser trimming as a contributory factor, and warrants quantity supply of inexpensive products because the semiconductor pressure sensor is provided on the surface opposite to the pressure-receiving surface thereof with output terminals, the output terminals are electrically connected to a connecting electrode formed on a flexible printed plate, and the output signal of the semiconductor pressure sensor is taken out to an external device through the medium of the printed plate.

In the pressure converter of this invention constructed as described above, since it suffices to connect electrically the output terminal of the semiconductor pressure sensor chip directly to the connecting electrode of the flexible printed plate, the electrical wiring necessary for-the pressure converter can be completed by one single step. In this invention, it becomes useless to adjust the output offset by trimming the thick-film resistor by laser as practiced conventionally. This point may be coped with by enhancing the uniformity of the process of production of the semiconductor pressure sensor thereby realizing production in a high yield of sensor chips which enjoy ideal bridge balance of piezo resistance and consequently obviate the necessity for trimming. To be specific, this invention makes positive use of the fact that so long as sensor chips are produced in a sufficiently high yield, the loss otherwise caused by performing laser trimming by the use of an expensive insulating substrate is decreased even if very few rejectables may be discarded.

Since this invention contemplates disposing the output terminal on the surface opposite to the pressure-receiving surface, the electric wires such as the lead line which are connected to the output terminal are not exposed to the pressure fluid under test and the extraction of the output signal is facilitated.

According to another embodiment of the invention, the flexible printed plate is disposed inside the pressing lid fitting part and the pressing lid is adapted to press the semiconductor pressure sensor against the outer wall surface of the fluid pipe through the medium of a spacer, and a platelike elastic member interposed between the pressing lid and the spacer and adapted to absorb the ununiform state of the pressing pressure arising from the fitting of the pressing lid.

The pressure converter of this invention enables the ununiformity of the pressing force suffered to occur when the pressing lid is fit into the pressing lid fitting part to be absorbed by the platelike elastic member and allows the semiconductor pressure sensor to be pressed with a uniform and proper pressing force because the sensor accommodating chamber accommodates therein the spacer and the platelike elastic member in conjunction with the semiconductor pressure sensor and the flexible printed plate and the semiconductor pressure sensor is pressed by the pressing lid fit into the pressing lid fitting part through the medium of the platelike elastic member and the spacer. As a result, the strain produced inside the semiconductor pressure sensor is reduced, the offset voltage of the output is decreased, and the linearity is improved. Further, the fact that a uniform pressing force is exerted between the semiconductor pressure sensor and the annular elastic member brings about an effect of precluding the occurrence of such adverse phenomena as leakage of liquid due to uneven pressure.

As claimed the invention proposes also a method for the production of a semiconductor pressure sensor having a sensor substrate including a depressed part in conjunction with a diaphragm part and a gauge resistor allowed to vary the magnitude thereof proportionately to the deformation of the diaphragm part, which method comprises the steps of:
- selectively etching a semiconductor wafer to a prescribed depth to form a plurality of depressed parts,
- forming a dielectric protective film at least on said depressed parts,
- separating said etched wafer coated with said dielectric protective film into chips to form said sensor substrate of said pressure sensor in conjunction with said diaphragm part, the pressure-receiving surface of said diaphragm part being formed by the depressed part coated with said dielectric protective film, such that the sensor substrate is prevented from being smeared with the pressure medium under test and, at the same time, electrically insulated from said pressure medium, by said dielectric protective film.

The method of this invention for the production of a semiconductor pressure sensor is capable of inexpensively mass-producing semiconductor pressure sensors possessing ideal temperature characteristics and electrical insulating property because the dielectric protective film can be formed by such a method as the plasma CVD which is generally used in the process for the production of semiconductor integrated circuits.

Particularly, the method of this invention for the production of a semiconductor pressure sensor adopts a silicon oxynitride film as the dielectric protective film, during the formation of this film, controls the property of the produced silicon oxynitride film by varying the flow rate ratio of plurality of reaction gases.

It is thus possible to obtain semiconductor pressure sensors of more stabilized temperature properties.

The physical properties such as internal stress of the produced silicon oxynitride film can be controlled by varying the flow rate ratio of the reaction gases (SiH₄, N₂ and N₂O) used in the CVD method. Thus, the film can be vested with a quality so as to enhance the properties of the pressure sensor. Since the silicon oxynitride film possesses hydrophilicity, it manifests affinity for the pressure medium which is in the form of a water soluble fluid. Therefore, the silicon oxynitride coated etched parts (depressions) avoid collecting stagnating air in their bottoms. Thus, the determination aimed at can be attained with high accuracy.
Fig. 1 is a perspective view of an embodiment of a pressure converter according to this invention,
Fig. 2 is a cross section taken through Fig. 1 along the line II-II,
Fig. 3 is a cross section illustrating another embodiment similarly to Fig. 2,
Fig. 4 is an exploded perspective view of an assembly as still another embodiment,
Fig. 5 is a cross section taken through Fig. 4 along the line V-V,
Fig. 6 is a cross section illustrating yet another embodiment similarly to Fig. 5,
Fig. 7 is a cross section of a semiconductor pressure sensor according to this invention, and
Figs. 8A to 8F are longitudinal cross sections jointly illustrating a process for the production of a semiconductor pressure sensor according to this invention.

In the pressure converter of this invention, since the semiconductor pressure sensor chip has the pressure-receiving surface thereof coated with a dielectric protective film, it is not directly exposed to the pressure fluid under test and is prevented from being stained with alkali metals (such as Na⁺ and K⁺) and alkaline earth metals (such as Ca²⁺) which are contained in the pressure fluid.

The substances which are effectively usable as a material for the dielectric protective film include fluorine type resins such as PFA (ethylenetetrafluorideperfluoroalkylvinyl/ ether copolymer resin), FEP (ethylene tetrafluoride-propylene hexafluoride copolymer resin), ETFE (ethylene tetrafluoride-ethylene copolymer resin), ECTFE (ethylene chlorotrifluoride-ethylene copolymer resin), PTFE (ethylene tetrafluoride resin), PCTFE (ethylene chlorotrifluoride resin), PVDF (vinylidene fluoride resin), and VDF (vinyl fluoride resin), metal oxides such as tantalum oxide (Ta₂O₃) and alumina (Al₂O₃), and silicon compounds such as silicon nitride film (SiN_{y}) and silicon oxynitride film (SiOₓN_{y}), for example. From the standpoint of procedure of production, such silicon compounds as silicon nitride film (SiN_{y}) and silicon oxynitride film (SiOₓN_{y}) are particularly useful. These silicon compounds excel in adaptability for mass production because they allow a desired film to be formed as superposed on a multiplicity of elements all at once by a batch process selected from among the techniques generally used for the formation of a thin film such as the plasma CVD (chemical vapor-phase deposition) method, the thermal CVD method, and the sputtering method. Since this dielectric protective film retains its physical properties stably and excels in electrical insulating property and imperviousness to ions, it is capable of infallibly insulating electrically the semiconductor pressure sensor chip and the pressure fluid for test from each other and stabilizing the sensor properties.

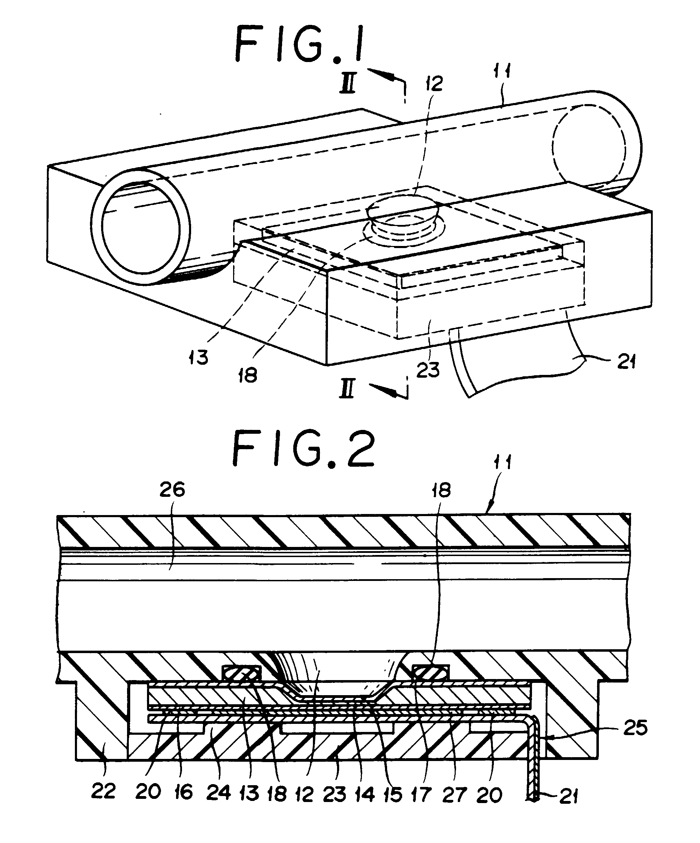

Fig. 1 is a perspective view illustrating the appearance of a disposable pressure converter as one embodiment of this invention and Fig. 2 is a cross section taken through Fig. 1 along the line II-II. A pressure detecting hole 12 is formed in the lateral wall of a cylindrical fluid pipe 11 and a semiconductor pressure sensor chip (hereinafter referred to as "sensor chip")13 is disposed on the outer wall surface of the fluid pipe 11 as faced to the pressure detecting hole 12. This sensor chip 13 is made of a semiconductor material such as, for example, silicon (Si). The central part of the sensor chip 13 is selectively etched to a prescribed depth from the rear side (upper side in the diagram) to form a thin-film diaphragm part 14. The rear surface side of the sensor chip 13 including the diaphragm part 14 is coated with a dielectric protective film such as, for example, a silicon oxynitride film 15. The front surface side of the sensor chip 13 is coated with a dielectric film such as, for example, a silicon oxynitride film 16. Optionally, the dielectric film may be formed of silicon oxide or silicon nitride, for example. A groove part 17 is formed in the periphery of the pressure detecting hole 12 of the fluid pipe 11. An 0-ring 18 is inserted in the groove part 17. The gap between the outer wall surface of the fluid pipe 11 and the rear surface side of the sensor chip 13 is watertightly sealed with this 0-ring 18 such as silicone rubber.

On the front surface (lower surface in the diagram) of the diaphragm part 14 of the sensor chip 13, a gauge resistor (not shown) made of an impurity-diffused layer is formed. This gauge resistor is electrically connected through the medium of a metallic lead (such as Aℓ, Au, Cu and Cr; not shown) to an output terminal (not shown) formed in the peripheral part of the sensor chip 13. Below the sensor chip 13, a flexible circuit 21 provided with a connecting electrode (contact pad) 20 is disposed as opposed to the output terminal of the sensor chip 13. The connecting electrode 20 are electrically connected to the output terminal by being soldered thereto.

On the outer wall surface of the fluid pipe 11, a pressing lid fitting part 22 is projectingly formed in such a manner as to encircle the sensor chip 13. A pressing lid 23 is fit upwardly in the diagram into the pressing lid fitting part 22. The flexible circuit 21 is led out through an outlet hole 25 formed in the pressing lid 23. On the inner surface of the pressing lid 23, a protuberant part 24 is formed as opposed to the groove part 17. In consequence of the fitting of the pressing lid 23 into the pressing lid fitting part 22, the protuberant part 24 imparts pressing force of severally suitable magnitudes between the flexible printed plate 21 and the sensor chip 13 and between the O-ring 18 and the sensor chip 13. No photovoltaic effect can be produced because the light impinging upwardly on the lower surface is intercepted by the copper foil 27 on the flexible substrate 21 and consequently prevented from reaching the sensor chip 13.

The protuberant part 24 is preferable, as illustrated in the diagrams, to be larger than the width of the groove part 17 in which the 0-ring 18 is inserted and to be adapted so as to exert pressure over the opposite sides of the groove part 17. The problem of the semiconductor sensor chip 13 being strained based on shearing stress to the pressure sensor chip and being warped by a difference in thermal expansion coefficient is eliminated by the pressure which is exerted on the pressure sensor chip from the side opposite thereto on the opposite faces of the O-ring.

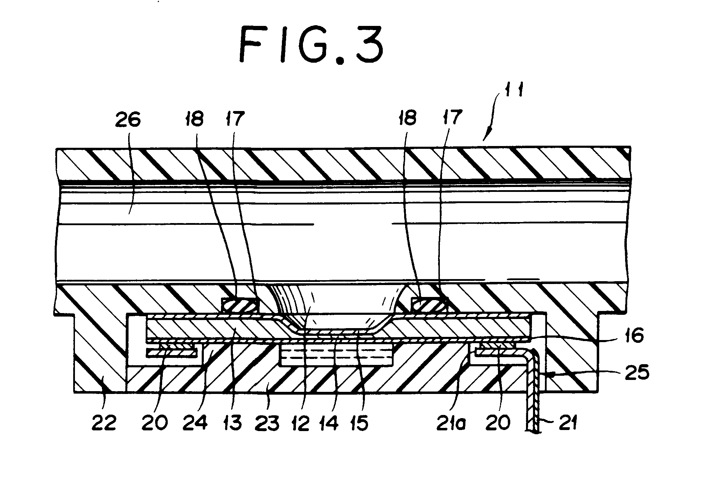

When the pressing lid 23 is vested with a light-intercepting function as illustrated in Fig. 3, a through hole 21a is formed in the central part of the flexible circuit 21 for the purpose of preventing the protuberant part 24 of the pressing lid 23 from contacting the flexible circuit 21 and enabling the protuberant part 24 to contact the sensor chip 13 directly and imparting pressing force of suitable magnitude between the 0-ring 18 and the sensor chip 13.

In the pressure converter of this embodiment, a pressure fluid 26 for test such as body fluid for transfusion flows inside the fluid pipe 11, the pressure of the pressure fluid 26 deforms the diaphragm 14 of the sensor chip 13, and the output signal namely the magnitude of resistance change of the gauge resistor (not shown) varies proportionately to this deformation of the diaphragm 14. Thus, the determination of the pressure of the pressure fluid 26 is accomplished by transmitting this output signal to an external display or recording equipment through the medium of the flexible circuit 21.

In the pressure converter of the present embodiment, since the diaphragm part 14 of the sensor chip 13 is coated with the silicon oxynitride film 15, the sensor chip 13 cannot directly contact the pressure fluid 26 and consequently the sensor chip 13 cannot be soiled with alkali metals (such as Na⁺ and K⁺) and alkaline earth metals (such as Ca²⁺), for example. Further, since this pressure converter avoids interposing the pressure transmitting medium of the form of gel between the sensor chip 13 and the pressure fluid 26 for test unlike the conventional counter type and consequently precludes perfectly the possibility of entailing such adverse phenomena as loss of pressure due to occurrence of bubbles in the pressure transmitting medium or damping in the process of pressure transmission, it allows accurate detection of a pressure waveform free from distortion or loss of sharpness.

Further, the pressure converter can be sealed infallibly and enabled to operate with enhanced reliability because the 0-ring kept pressed with suitable force by the pressing lid 23 watertightly seals the gap between the outer wall surface of the fluid pipe 11 and the sensor chip 13 and additionally because the pressure converter has no component parts joined with adhesive agent. Actually in an experiment, the pressure converter showed absolutely no discernible sign of leakage of the pressure fluid 26 when pressure exceeding about 300 mmHg was applied to the pressure fluid 26 inside the pressure converter.

In the pressure converter of this embodiment, since the output terminals 19 of the sensor chip 13 are disposed as opposed to the connecting electrode 20 of the flexible circuit 21, the electrical connecting required at all for the pressure converter can be completed by one single step of connecting the opposed components by soldering. This pressure converter allows a generous cut in the number of component parts as compared with the conventional pressure converter because the parts participating in the electrical connection are only the sensor chip 13 and the flexible printed plate 21. In the process for production of sensors, sensor chips 13 of ideally balanced bridge resistance can be obtained in a high yield exceeding about 90%. Most of these sensor chips 13 thus mass produced, therefore, do not require trimming with a laser beam. The loss arising from discarding the rejectable products is smaller than the expense which is otherwise incurred in trimming bridge resistors by the conventional method.

The embodiment cited above has been depicted as having the entire rear surface of the sensor chip 13 coated with the silicon oxynitride film 15. This silicon oxynitride film 15 is required to be formed only in a region which is destined to contact the pressure fluid 26 under test. Of course, the dielectric protective film may be a silicone nitride film instead of the silicon oxynitride film 15.

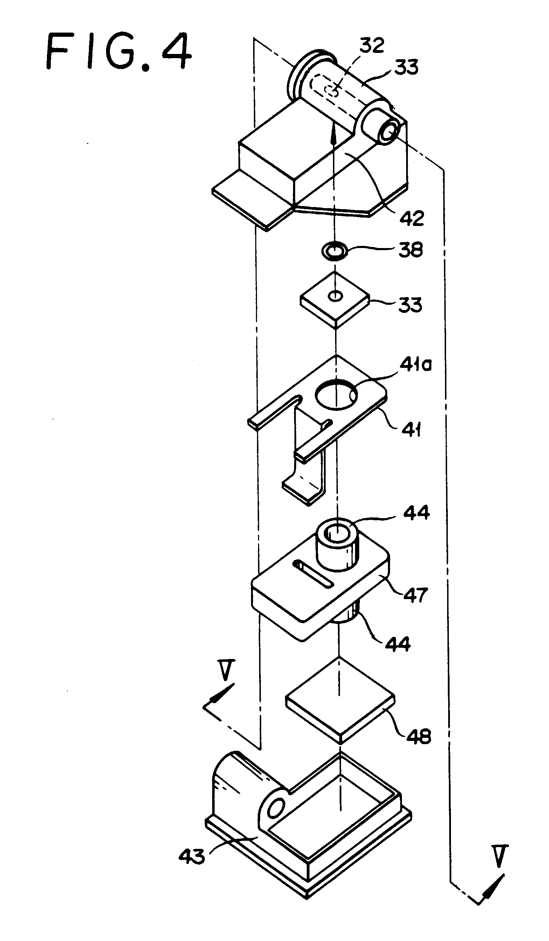

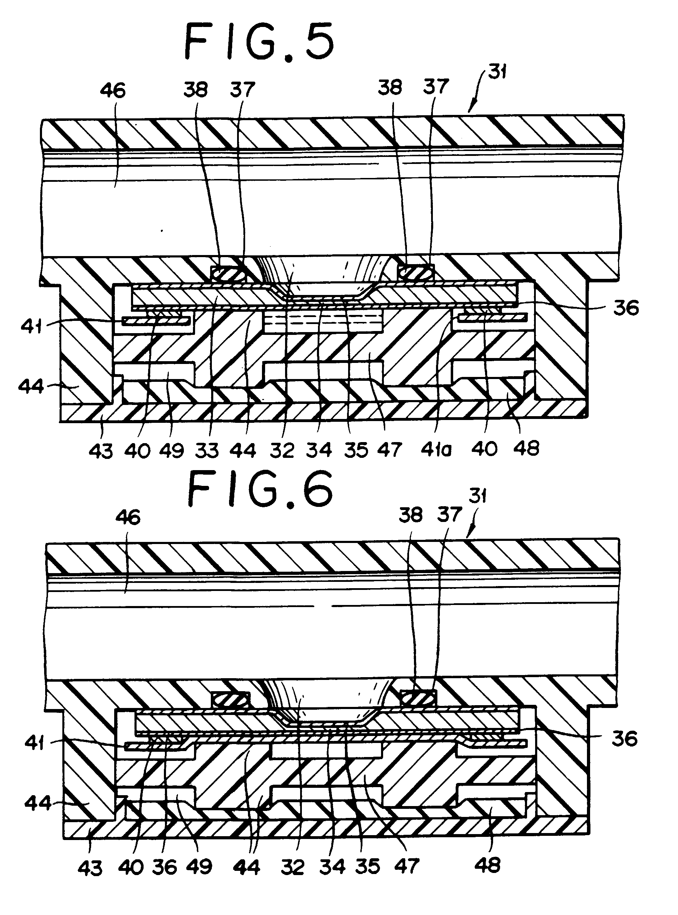

Fig. 4 is an exploded perspective view illustrating the positional relation of component parts of the pressure converter as another embodiment of this invention and Fig. 5 is a cross section taken through Fig. 5 along the line V-V to illustrate the construction obtained in consequence of assembly.

A spacer 47 and a rubber piece 48 are nipped between a semiconductor pressure sensor 33 and a pressing lid 43. Protuberant parts 44 are formed one each on the upper and lower sides of the spacer 47 as opposed to grooved parts 37. In consequence of the fitting of the pressing lid 43 into a pressing lid fitting part 42, the protuberant part 44 pierces a through hole 41a of a flexible circuit 41 and directly presses the semiconductor pressure sensor 33 and, as a result, imparts pressing force of suitable magnitude between the semiconductor pressure sensor 33 and an 0-ring 38. Since this pressing force is produced by the fitting of the pressing lid 43 into the pressing lid fitting part 42 as described above, the possibility of uneven pressing force being exerted on the semiconductor pressure sensor 33 will ensue, depending on the condition of fabrication of the two component parts involved herein. The rubber piece 48 fastened with adhesive agent to the pressing lid 43 and eventually nipped between the pressing lid 43 and the spacer 47 plays the role of absorbing the uneven state of the pressing force by virtue of its softness and imparting substantially uniform pressing force to the semiconductor pressure sensor 33.

In the pressure converter of this embodiment, since the rubber piece 48 is nipped between the pressing lid 43 and the spacer 47 and consequently enabled to absorb gently the uneven state of the pressing force generated in consequence of the fitting of the pressing lid 42 into the pressing lid fitting part 42, the semiconductor pressure sensor 33 can be pressed with the pressing force of substantially uniform and suitable magnitude through the medium of the spacer 47. As a result, the offset voltage which is liable to occur in the pressure converter illustrated in Figs. 1 to 3 can be decreased, the linearity improved, and the quality enhanced.

The semiconductor pressure sensor 33, the flexible circuit 41 and the spacer 47 are shaped so as to conform to the inner wall of a sensor accommodating chamber 49 formed inside the pressing lid fitting part 42 and, therefore, can be inserted with ample room left. Thus, these two components are stowed at suitable positions within the sensor accommodating chamber 49. The semiconductor pressure sensor 33 is preparatorily joined by soldering to the flexible circuit 41, but a diaphragm 34 of the semiconductor pressure sensor 33 and a pressure detecting hole 32 of a fluid pipe 31 has to be assembled as suitably positioned relative to each other.

In the embodiment described thus far, for example, the pressing lid 43 is vested with a light-intercepting function and the flexible circuit 41 is provided with the through hole 41a so that the the protuberant part 44 of the spacer 47 directly presses the semiconductor pressure sensor 33 and imparts pressing force of suitable magnitude between the 0-ring 38 and the semiconductor pressure sensor 33. When the pressing lid 43 is not preferable to be vested with the light-intercepting function, the flexible circuit is vested with the light-intercepting function and the protuberant part 44 of the spacer 47 is adapted, as illustrated in Fig. 6, to exert indirect pressure to the semiconductor pressure sensor 33 through the medium of the flexible circuit 42. Otherwise, a light-intercepting sheet or light-intercepting layer may be interposed between the flexible circuit 41 and the pressing lid 43. In the embodiment described above, the spacer is provided with protuberant parts 44 one each on the upper and lower side thereof. Optionally, the protuberant part 44 on the lower side may be shaped in a varying pattern to suit particular need or the protuberant parts 44 may be totally omitted.

In Figs. 4 to 6, the reference numerals which are sums of the reference numerals of Figs. 1 to 3 plus 20 represent the same component parts as those of Figs. 1 to 3.

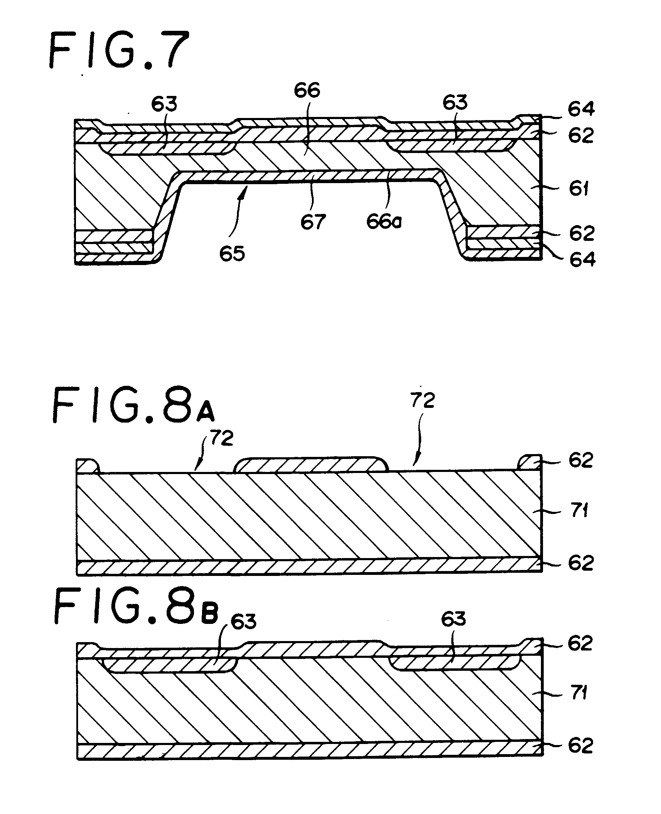

Fig. 7 is a cross section representing the construction of a semiconductor pressure sensor chip as another embodiment of this invention. The semiconductor pressure sensor of this embodiment is provided with a sensor substrate 61 formed of silicon, for example. In this sensor substrate 61, a depressed part 65 of prescribed depth is formed in conjunction with a diaphragm part 66. The rear surface of this diaphragm part 66, or the part forming the bottom part of the depressed part 65, serves as a pressure-receiving surface 66a. This pressure-receiving surface 66a deforms proportionately to the pressure of the pressure fluid. A plurality of gauge resistors 63 are formed on the surface opposite the pressure-receiving surface 66a of the diaphragm part 66. These gauge resistors 63 are formed of an impurity diffused layer, for example, and are adapted to vary magnitude of resistance proportionately to the deformation of the diaphragm part 66. These gauge resistors 63 are electrically connected to metallic leads (such as Aℓ, Au, Cu and Cr; not shown), so that the changes in their magnitudes of resistance may be taken out as output signals to an external equipment. The front surface of the sensor substrate 61 and the rear surface thereof except for the depressed part 65 are coated with a two-layer insulating film consisting of a silicon oxide (SiO₂) layer 62 and a silicon nitride (SiN_{y}) layer 64. Further, the rear surface side of the sensor substrate 61 including the pressure-receiving surface 66a of the diaphragm 66 is coated with a dielectric protective film such as, for example, a silicon oxynitride film 67 having a prescribed thickness.

In the semiconductor pressure sensor of this embodiment, the diaphragm part 66 deforms proportionately to the pressure of the pressure fluid under test and the magnitudes of resistance of the gauge resistors 63, namely the output signals, vary proportionately to the degree of this deformation of the diaphragm part 66. Thus, the determination of pressure of the pressure fluid can be attained as aimed at.

In the semiconductor pressure sensor of the present embodiment, the sensor chip and the pressure medium under test are electrically insulated from each other and, at the same time, the diaphragm part 66 is prevented from being stained with the pressure medium under test because the pressure-receiving surface 66a of the diaphragm 66 is coated with the dielectric protective film formed of the silicon oxynitride film 67. This silicon oxynitride film 67 is capable of substantially eliminating possible difference in thermal expansion coefficient from the diaphragm part 66 made of silicon and, therefore, is incapable of exerting any adverse effect on the deforming action of the diaphragm part 66.

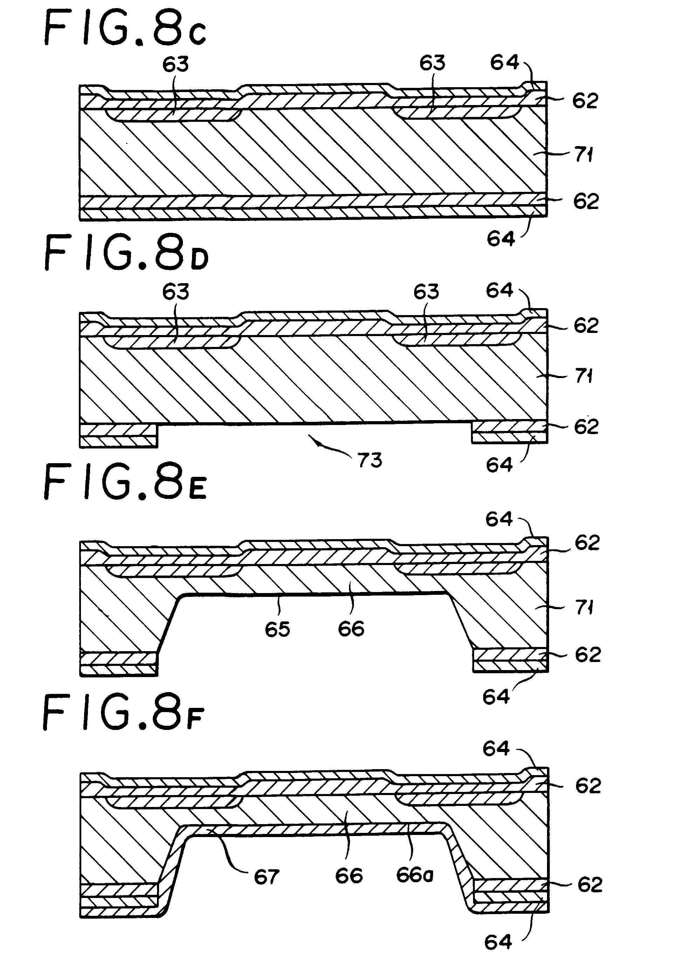

Now, the method for the production of the semiconductor pressure sensor embodying this invention will be described below with reference to Figs. 8A to 8F. First, a silicon wafer (about 300 to about 400 µm in thickness) 71 before separation into chips is thermally oxidized to form silicon oxide films 62 about 0.5 µm in thickness one each on the front and rear surface of the silicon wafer as illustrated in Fig. 8A. Subsequently, the silicon oxide film 62 is selectively removed by photolithographic etching to form window parts 72 for diffusion.

Then, boron is ion injected as an impurity in a rate of 3.1 x 10¹⁵ cm⁻² into the silicon wafer 71 through the windows opened in the silicon oxide film 62 as illustrated in Fig. 8B and the boron is drive-in diffused at 1,000°C for 90 minutes to give rise to a gauge resistor 63 formed of an impurity-diffused layer. While the drive-in diffusion is in process, a silicon oxide film is formed in the window part 72.

Subsequently, silicon nitride (SiN_{y}) films 0.2 µm in thickness are formed one each on the front and rear surface of the silicon oxide film 62 as illustrated in Fig. 8C, by the CVD process at a temperature (700° to 950°C) such as to avoid altering the gauge resistor 63 made of the impurity-diffused layer.

Then, the silicon nitride film 64 and the silicon oxide film 62 on the rear surface of the sensor substrate 61 are selectively etched as illustrated in Fig. 8D to form a window part 73 intended for formation of a diaphgram 66. Subsequently, the silicon wafer 71 is etched from the rear surface side of the sensor substrate 61 through the window part 73 opened with the silicon nitride film 64 and the silicon oxide film 62 each serving as a mask, to give rise to the depressed part 65 and the diaphragm part 66.

Now, on the rear surface side of the silicon wafer 71 including the pressure-receiving surface 66a of the diaphragm part 66, a silicon oxynitride film (SiOₓN_{y}) 67 having a thickness of 0.5 µm is formed as illustrated in Fig. 8F by the plasma CVD process. The conditions for the formation of this film are 450°C in substrate temperature, 400 W in high-frequency power, 15 CCM of Monosilane (SiH₄), 200 SCCM of nitrogen (N₂), and 35 SCCM of laughing gas (N₂O) respectively in gas flow volume, 0.45 Torr in operating pressure, and 0.11 µm/min. in film-deposition rate.

Since the silicon oxynitride film 67 enables the quality thereof such as inner stress to be controlled by varying the flow rate ratio [N = N₂/(N₂ + N₂O)] of the reaction gases (N₂ and N₂O) flowing inside the reaction chamber during the deposition of the film, it allows production of the pressure sensor which excels such as in the zero point temperature characteristic. From the standpoint of the ability of the film to intercept ions and the inner stress, the value of the N which warrants production of a film capable of serving as a suitable protective film for the pressure sensor is in the range between 0.835 and 0.865. In the present embodiment, the most desirable results evinced by a zero point temperature drift of not more than ±0.1% F.S./°C are obtained when N = 0.851. The thickness and area of the diaphragm part 66 are fixed by the range of pressure expected to be determined and the optimum gas flow ratio and the optimum thickness of the silicon oxynitride film 67 are varied by the thickness and area mentioned above.

The steps of the formation of a contact hole, the formation of an electrode, and the dicing which follow the formation of the silicon oxynitride film 67 carried out as described above are the same as those involved in the conventional method and, therefore, will be omitted from the following description.

The pressure converter of the present embodiment can be produced by the batchwise process generally adopted in the- ordinary production of a semiconductor integrated circuit because the silicon oxynitride film 67 is formed on the surface including the pressure-receiving surface 66a of the diaphragm part 66 as a means for preventing the sensor chip from being smeared with the pressure medium under test and, at the same time, establishing electrical insulation between the sensor chip and the pressure medium under test. Thus, semiconductor pressure sensors can be mass produced inexpensively.

The embodiment thus far cited represents a case in which the plasma CVD process is used in the formation of the silicon oxynitride film 67. Optionally, the film may be formed by some other process such as the thermal CVD process or sputtering process. The embodiment has been depicted as using the silicon oxynitride film as a dielectric protective film. Of course, some other suitable film such as the silicon nitride film may be used instead.

In the embodiment cited above, the dielectric protective film is formed on the entire rear surface including the depressed part 65 of the sensor substrate 61. This dielectric protective film is only required to coat at least the depressed part 65.

## Claims

1. A pressure converter comprising a semiconductor pressure sensor (13) including a sensor substrate, a diaphragm part (14) formed on said sensor substrate and adapted to deform proportionately to the pressure emanating from a pressure medium under test, a gauge resistor adapted to vary the magnitude of resistance thereof proportionately to the deformation of said diaphragm part, characterized by the fact that at least the pressure-receiving surface of said diaphragm part is coated with a silicon oxynitride protective film (15).

2. A pressure converter according to claim 1, wherein the opposite pressure-receiving surfaces of said diaphragm part (14) are coated with a protective film (16).

3. A pressure converter according to claim 1 or 2, further comprising a fluid pipe (11) adapted to allow flow of a pressure fluid therein, a pressure detecting hole (12) provided in the lateral wall of said fluid pipe (11), and wherein said pressure-receiving surface of said diaphragm part is disposed as opposed to said pressure detecting hole (12).

4. A pressure converter according to claim 3, which further comprises pressing means (22,23) for pressing the periphery of said pressure-receiving surface of said semiconductor pressure sensor (13) against the outer wall surface of said fluid pipe (11) and sealing means for watertightly sealing the gap intervening between the outer wall surface of said fluid pipe and said semiconductor sensor.

5. A pressure converter according to claim 4, wherein said pressing means is composed of a pressing lid fitting part (22) disposed projectingly in the periphery of said pressure detecting hole in said fluid pipe and a pressing lid (23) allowed to fit in said pressing lid fitting part, provided with a protuberant part, and adapted to press said semiconductor pressure sensor against the outer wall surface of said fluid pipe by virtue of said protuberant part (24).

6. A pressure converter according to claim 4 or claim 5, wherein said sealing means is composed of a groove part (17) formed in the outer wall surface of said fluid pipe in such a manner as to encircle said pressure detecting hole and an annular elastic member allowed to fit into said groove part and adapted to be pressed by said protuberant part of said pressing lid through the medium of said semiconductor pressure sensor.

7. A pressure converter according to any of claims to 6, wherein said semiconductor pressure sensor (13) is provided on the surface opposite to said pressure-receiving surface thereof with an output terminal, said output terminal is electrically connected to a connecting electrode formed on a flexible printed plate (21), and the output signal from said semiconductor pressure sensor (13) is taken out to an external device through the medium of said printed plate (21).

8. A pressure converter according to claims 5 and 7, wherein said flexible printed plate is disposed inside said pressing lid fitting part, said pressing lid is adapted to press said semiconductor pressure sensor against the outer wall Surface of said fluid pipe through the medium of a spacer (47), and a platelike elastic member (48) interposed between said pressing lid and said spacer and adapted to absorb the ununiform state of the pressing pressure arising from the fitting of said pressing lid.

9. A method for the production of a semiconductor pressure sensor having a sensor substrate (61) including a depressed part (65) in conjunction with a diaphragm part (66) and a gauge resistor (63) allowed to vary the magnitude thereof proportionately to the deformation of the diaphragm part (66), which method comprises the steps of:
- selectively etching a semiconductor wafer (71) to a prescribed depth to form a plurality of depressed parts (65),
- forming a dielectric protective film (67) at least on said depressed parts,
- separating said etched wafer (71) coated with said dielectric protective film (67) into chips to form said sensor substrate (61) of said pressure sensor in conjunction with said diaphragm part (66), the pressure-receiving surface of said diaphragm part being formed by the depressed part (65) coated with said dielectric protective film (67), such that the sensor substrate (61) is prevented from being smeared with the pressure medium under test and, at the same time, electrically insulated from said pressure medium, by said dielectric protective film (67).

10. A method according to claim 9, wherein said dielectric protective film is formed of a silicon oxynitride film (67), said semiconductor wafer (71) is swept with a plurality of reaction gases during the formation of said film, and the quality of said silicon oxynitride film (67) is controlled by varying the flow ratio of said reaction gases.

## Patentansprüche

1. Druckwandler, umfassend einen Halbleiterdrucksensor (13), der ein Sensorsubstrat enthält, einen Diaphragmateil (14), der auf dem Sensorsubstrat gebildet ist und in der Lage ist, sich proportional zu dem von einem in Untersuchung befindlichen Druckmedium herkommenden Druck zu deformieren, einen Meßwiderstand, dessen Widerstandsgröße sich proportional zur Deformation des Diaphragmateils ändern kann, dadurch **gekennzeichnet,** daß wengistens die Druckaufnahmefläche des Diaphragmateils mit einem Siliciumoxynitrid-Schutzfilm (15) überdeckt ist.

2. Druckwandler nach Anspruch 1, bei dem die gegenüberliegenden Druckaufnahmeflächen des Diaphragmateils (14) mit einem Schutzfilm (16) überdeckt sind.

3. Druckwandler nach Anspruch 1 oder 2, weiter umfassend, ein Fluidrohr (11), das die Strömung eines Druckfluids darin gestatten kann, ein Druckerfassungsloch (12), das in der Seitenwand des Druckrohrs (11) vorgesehen ist, und wobei die Druckaufnahmefläche des Diaphragmateils dem Druckerfassungsloch (12) gegenüberliegend vorgesehen ist.

4. Druckwandler nach Anspruch 3, der weiter ein Druckbeaufschlagungsmittel (22, 23) zur Druckbeaufschlagung der Druckaufnahmefläche des Halbleiterdrucksensors (13) gegen die Außenwandfläche des Fluidrohrs (11) und ein Abdichtmittel zur wasserdichten Abdichtung des Spalts umfaßt, der zwischen einer Außenwandfläche des Druckrohrs und dem Halbleitersensor auftritt.

5. Druckwandler nach Anspruch 4, bei dem das Druckbeaufschlagungsmittel aus einem Druckdeckel-Montageteil (22) besteht, das vorstehend im Umfang des Druckerfassungslochs im Druckrohr vorgesehen ist, und einem Druckdeckel (23), der in den Druckdeckel-Montageteil paßt, versehen mit einem vorstehenden Teil und in der Lage, den Halbleiterdrucksensor gegen die Außenwandfläche des Druckrohrs mittels des vorstehenden Teils (24) zu drücken.

6. Druckwandler nach Anspruch 4 oder Anspruch 5, bei dem das Dichtmittel aus einem Nutenteil (17) besteht, der in der Außenwandfläche des Fluidrohrs auf solche Weise gebildet ist, daß er das Druckerfassungsloch umgibt, und einem elastischen Ringglied, das in den Nutenteil passen kann und geeignet ist, durch den vorstehenden Teil des Druckdeckels mittels des Halbleiterdrucksensors druckbeaufschlagt zu werden.

7. Druckwandler nach einem der Ansprüche 1 bis 6, bei dem der Halbleiterdrucksensor (13) auf der Oberfläche gegenüberliegend der Druckaufnahmefläche mit einem Ausgangsanschluß versehen ist, der Ausgangsanschluß mit einer Anschlußelektrode elektrisch verbunden ist, die auf einer flexiblen Leiterplatte (21) gebildet ist, und das Ausgangssignal von dem Halbleiterdrucksensor (13) zu einer externen Vorrichtung mittels der Leiterplatte (21) abgenommen wird.

8. Druckwandler nach den Ansprüchen 5 und 7, bei dem die flexible Leiterplatte im Innern des Druckdeckel-Montageteils vorgesehen ist, der Druckbeaufschlagungsdeckel in der Lage ist, den Halbleiterdrucksensor gegen die Außenwandfläche des Druckrohrs mittels eines Abstandsstücks (47) und ein plattenartiges elastisches Glied (48) zu drücken, das zwischen dem Druckdeckel und dem Abstandsstück angeordnet ist und in der Lage ist, den nicht gleichmäßigen Zustand der von dem Anbringen des Druckdekkels auftretenden Festdruck zu absorbieren.

9. Verfahren zur Herstellung eines Halbleiterdrucksensors mit einem Sensorsubstrat (61), enthaltend einen vertieften Teil (65), zusammen mit einem Diaphragmateil (66) und einem Meßwiderstand (63), dessen Größe sich proportional zur Deformation des Diaphragmateils (66) ändern kann, welches Verfahren die Schritte umfaßt, daß:
- ein Halbleiterwafer (71) auf eine vorbestimmte Tiefe selektiv -zur Bildung einer Anzahl von vertieften Teilen (65) geätzt wird,
- ein dielektrischer Schutzfilm (67) wenigstens auf den vertieften Teilen gebildet wird,
- der geätzte Wafer (71), der mit dem dielektrischen Schutzfilm (67) überdeckt ist, in Chips aufgeteilt wird, um das Sensorsubstrat (61) des Drucksensors zusammen mit dem Diaphragmateil (66) zu bilden, wobei die Druckaufnahmefläche des Diaphragmateils durch den vertieften Teil (65), der mit dem dielektrischen Schutzfilm (67) überdeckt ist, gebildet wird derart, daß verhindert wird, daß das Sensorsubstrat (61) mit dem in Untersuchung befindlichen Druckmedium verschmutzt wird, und gleichzeitig durch den dielektrischen Schutzfilm (67) von dem Druckmedium elektrisch isoliert wird.

10. Verfahren nach Anspruch 9, bei dem der dielektrische Schutzfilm aus einem Siliciumoxynitridfilm (67) gebildet wird, der Halbleiterwafer (71) mit einer Anzahl von Reaktionsgasen während der Bildung des Films überstrichen wird und die Qualität des Siliciumoxynitridfilms (67) kontrolliert wird, indem das Strömungsverhältnis der Reaktionsgase geändert wird.

## Revendications

1. Convertisseur de pression comprenant un capteur de pression (13) à semi-conducteur comprenant un substrat de capteur, une partie diaphragme (14) formée sur ledit substrat de capteur et adaptée pour se déformer proportionnellement à la pression émanant d'un milieu sous pression soumis à une mesure, une résistance de mesure adaptée pour modifier la valeur ohmique de cette résistance proportionnellement à la déformation de ladite partie diaphragme, **caractérisé** par le fait qu'au moins la surface réceptrice de pression de ladite partie diaphragme est revêtue d'un film protecteur (15) en oxynitrure de silicium.

2. Convertisseur de pression selon la revendication 1, dans lequel les surfaces opposées, réceptrices de pression, de ladite partie diaphragme (14) sont revêtues d'un film protecteur (16).

3. Convertisseur de pression selon la revendication 1 ou 2, comprenant, en outre, un conduit (11) de fluide adapté pour y permettre l'écoulement d'un fluide sous pression, un trou (12) de détection de pression ménagé dans la- paroi latérale dudit conduit (11) de fluide, et dans lequel ladite surface réceptrice de pression de ladite partie diaphragme est disposée en face dudit trou (12) de détection de pression.

4. Convertisseur de pression selon la revendication 3, comprenant, en outre, un moyen de pression (22, 23) pour presser la périphérie de ladite surface réceptrice de pression dudit capteur de pression (13) à semi-conducteur contre la surface extérieure de la paroi dudit conduit (11) de fluide et un moyen d'étanchéité pour obturer de façon étanche l'interstice compris entre la surface extérieure de la paroi dudit conduit de fluide et ledit capteur à semi-conducteur.

5. Convertisseur de pression selon la revendication 4, dans lequel ledit moyen de pression est composé d'une partie (22) de montage de couvercle de pression disposée, de façon saillante, dans la périphérie dudit trou de détection de pression dudit conduit de fluide et un couvercle de pression (23) qui peut être emboîté dans ladite partie de montage de couvercle de pression, pourvue d'une partie protubérante, et adapté pour presser ledit capteur de pression à semi-conducteur contre la surface extérieure de la paroi dudit conduit de fluide à l'aide de ladite partie protubérante (24).

6. Convertisseur de pression selon la revendication 4 ou la revendication 5, dans lequel ledit moyen d'étanchéité est composé d'une partie formant rainure (17) ménagée dans la surface extérieure de la paroi dudit conduit de fluide de manière à entourer ledit trou de détection de pression et d'un élément élastique annulaire pouvant être encastré dans ladite rainure et adapté pour être soumis à une pression par la partie protubérante dudit couvercle de pression par l'intermédiaire dudit capteur de pression à semi-conducteur.

7. Convertisseur de pression selon l'une quelconque des revendications 1 à 6, dans lequel ledit capteur de pression (13) à semi-conducteur est pourvu d'une borne de sortie sur la surface opposée à sa surface réceptrice de pression précitée, ladite borne de sortie étant connectée électriquement à une électrode de connexion formée sur une plaque imprimée flexible (21) et le signal de sortie en provenance du capteur de pression (13) à semi-conducteur étant extrait vers un dispositif extérieur par l'intermédiaire de ladite plaque imprimée (21).

8. Convertisseur de pression selon les revendications 5 et 7, dans lequel ladite plaque imprimée flexible est disposée à l'intérieur de ladite partie de montage de couvercle de pression, ledit couvercle de pression étant adapté pour presser ledit capteur de pression à semi-conducteur contre la surface extérieure de la paroi dudit conduit de fluide par l'intermédiaire d'un élément d'espacement (47), et un élément élastique (48) analogue à une plaque, interposé entre ledit couvercle de pression et ledit élément d'espacement et adapté pour absorber l'état non-uniforme de la pression provenant du montage dudit couvercle de pression.

9. Procédé pour la fabrication d'un capteur de pression à semi-conducteur comportant un substrat (61) de capteur comprenant une partie en creux (65) conjointement avec une partie diaphragme (66) et une résistance de mesure (63) dont la valeur peut varier proportionnellement à la déformation de la partie diaphragme (66), ce procédé comprenant les étapes consistant :
- à attaquer sélectivement une tranche (71) de semi-conducteur jusqu'à une profondeur prescrite de manière à former une pluralité de parties en creux (65),
- à former un film diélectrique protecteur (67) au moins sur lesdites parties en creux,
- à séparer ladite tranche attaquée (71) revêtue dudit film diélectrique protecteur (67) en puces de manière à former ledit substrat (61) dudit capteur de pression conjointement avec ladite partie diaphragme (66), la surface réceptrice de pression de ladite partie diaphragme étant formée par la partie en creux (65) revêtue dudit film diélectrique protecteur (67), de telle sorte que le substrat (61) du capteur ne soit pas maculé par le fluide sous pression soumis à une mesure, en même temps, soit isolé électriquement dudit milieu sous pression par ledit film protecteur diélectrique (67).

10. Procédé selon la revendication 9, dans lequel on forme ledit film diélectrique protecteur avec un film (67) d'oxynitrure de silicium, on balaye la tranche (71) de semi-conducteur avec une pluralité de gaz de réaction pendant la formation dudit film, et on agit sur la qualité dudit film (67) d'oxynitrure de silicium en faisant varier le rapport de débit desdits gaz de réaction.
